# EUROPEAN PATENT SPECIFICATION

(11) **EP 2 198 296 B1**
(45) Date of publication and mention of the grant of the patent: **11.11.2015**
(21) Application number: 08783436.2
(22) Date of filing: 05.09.2008
(51) Int. Cl.: C12Q 1/68, G01N 33/48, G01N 30/00, G01N 33/574

(54) **METHOD OF USING TUMOUR RNA INTEGRITY TO MEASURE RESPONSE TO CHEMOTHERAPY IN CANCER PATIENTS**
VERFAHREN ZUR MESSUNG DES ANSPRECHENS AUF CHEMOTHERAPIE BEI KREBSPATIENTEN UNTER VERWENDUNG VON TUMOR-RNA-INTEGRITÄT
PROCÉDÉ D'UTILISATION DE L'INTÉGRITÉ D'ARN TUMORAL POUR MESURER LA RÉPONSE À LA CHIMIOTHÉRAPIE CHEZ DES PATIENTS ATTEINTS DE CANCER

(30) Priority: 05.09.2007 US 935874 P; 06.09.2007 US 935903 P
(43) Date of publication of application: 23.06.2010
(73) Proprietor: Laurentian University, Sudbury, Ontario P3E 2C6 (CA)
(72) Inventor: PARISSENTI, Amadeo, Mark, Sudbury, ON P3E 1Z7 (CA); GUO, Baoqing, Sudbury, ON P3E 1Z7 (CA)
(74) Representative: Turner, Rhiannon Rosalind
(86) International application number: PCT/CA2008/001561
(87) International publication number: WO 2009/030029

(56) References cited:
- EP-A1- 1 772 522
- US-A1- 2006 166 231
- BALATSOS N A A ET AL: "Drug action on poly(A) polymerase activity and isoforms during U937 cell apoptosis", JOURNAL OF EXPERIMENTAL AND CLINICAL CANCER RESEARCH, ROME, IT, vol. 20, no. 1, 1 January 2001 (2001-01-01), pages 63-69, XP009145046, ISSN: 0392-9078
- OGSTON KEITH N ET AL: "A new histological grading system to assess response of breast cancers to primary chemotherapy: Prognostic significance and survival.", BREAST, vol. 12, no. 5, October 2003 (2003-10), pages 320-327, XP002624792, ISSN: 0960-9776
- CAREY LISA A ET AL: "Telomerase activity and prognosis in primary breast cancers", JOURNAL OF CLINICAL ONCOLOGY, vol. 17, no. 10, October 1999 (1999-10), pages 3075-3081, XP002624793, ISSN: 0732-183X
- PARISSENTI A M ET AL: "Relationship of tumor RNA integrity to clinicopathologic parameters associated with epirubicin/docetaxel chemotherapy", INTERNET CITATION, 7 September 2007 (2007-09-07), pages 1-4, XP003026532, Retrieved from the Internet: URL:http://www.asco.org/ASCOv2/Meetings/Ab stracts?&vmview=abst_detail_vie w&confID=52&abstractID=40345 [retrieved on 2010-10-01]
- SCHROEDER ANDREAS ET AL: "The RIN: an RNA integrity number for assigning integrity values to RNA measurements", BMC MOLECULAR BIOLOGY, BIOMED CENTRAL LTD, GB, vol. 7, no. 1, 31 January 2006 (2006-01-31), page 3, XP021014979, ISSN: 1471-2199, DOI: DOI:10.1186/1471-2199-7-3
- WONG ET AL.: 'Reduced Plasma RNA Integrity in Nasopharyngeal Carcinoma Patients' CLINICAL CANCER RESEARCH vol. 12, no. 8, 2006, pages 2512 - 2516, XP003026531
- PARISSENTI ET AL.: 'Relationship of tumor RNA integrity to clinicopathologic parameters associated with epirubicin/docetaxel chemotherapy' BREAST CANCER SYMPOSIUM no. ABSTRACT 107, 07 September 2007 - 08 September 2007, XP003026532

## Description

### Field of the Invention

The present invention relates to a method of determining tumour response to chemotherapy by comparing the RNA integrity of tumour cells before, during and after chemotherapy.

### Background of the Invention

Cancer is the uncontrolled malignant growth of cells. In a process called metastasis, cancerous cells can spread from their site of origin to distant sites within the body, via the lymphatic and/or circulatory systems. Metastasis is the leading cause of death in humans with cancer (Bockhorn, M. et al., Lancet Oncol. 8 (2007) 444-448.)

There are a number of treatments which are used to treat or control cancer, including surgery, radiation therapy and chemotherapy. Surgery and radiation therapy are typically used to remove non-metastatic cancerous tumours (abnormal growths composed of cancerous cells). However, the presence of metastatic cancer necessitates the use of systemic chemotherapy regimens to combat the growth of primary tumours (before or after surgery) and secondary tumours throughout the body. In breast cancer, effective systemic chemotherapy agents include the anthracyclines (typically doxorubicin or epiuribicin), taxanes (paclitaxel or docetaxel), nucleoside analogs (5-fluorouracil), and alkylating agents (cyclophosphamide) (Parissenti, A.M. et al. Anticancer Drugs 18 (2007) 499-523). Anthracyclines disrupt the uncoiling of DNA by topoisomerase II ("topo II"), intercalate between DNA strands, and cause DNA lesions, thereby interfering with DNA replication in rapidly dividing tumour cells. Taxanes, on the other hand, block the depolymerization of microtubules, resulting in an arrest of the cell cycle at mitosis and the subsequent induction of apoptosis (Distefano, M. et al., Int. J. Cancer 72 (1997) 844-850; Moos, P.J. et al., Proc. Natl. Acad. Sci. U.S.A 95 (1998) 3896-3901). The nucleoside analog 5-fluorouracil blocks the conversion of dUMP into dTMP, while the alkylating agent cyclophosphamide forms covalent bonds with DNA (Parker, W.B. et al., Pharmacol Ther. 48 (1990) 381-395; Bignold, L.P., Anticancer Res. 26 (2006) 1327-1336). These latter two drugs disrupt DNA replication in rapidly dividing cells at S phase (Zijlstra, J.G. et al. Oncol. Tumor Pharmacother. 7 (1990) 11-18; Richardson, D.S. et al., Blood Rev. 11 (1997) 201-223; Capranico, G. et al., Chem. Biol. Interact. 72 (1989) 113-123; Chazard,M. et al., Bull. Cancer 81 (1994) 173-181).

Most drugs that are used in chemotherapy are highly cytotoxic, and destroy both healthy normal cells (particularly if they are rapidly dividing) and cancerous cells. As such, chemotherapy drugs cause significant side effects, such as immunosupression, nausea and vomiting, and cardiotoxicity. These side effects can have a significant negative effect on the patient's quality of life.

Tumour response to chemotherapy agents can vary widely between patients, due to the presence of drug resistance mechanisms in some patients that block drug efficacy. Drug resistance can be "intrinsic" (i.e. pre-exist in the tumour) or "acquired" through continued exposure to chemotherapy agents. A number of mechanisms have been identified which play a role in reduced responsiveness of tumour cells to chemotherapy agents *in vitro.* For the anthracyclines and taxanes, these include the overexpression of drug transporters (e.g. P-glycoprotein) and the multidrug resistance protein, the downregulation of topoisomerase II α, mutations in the cell cycle regulator protein p53, the increased synthesis of thymidylate synthase or the drug-conjugating enzyme glutathione-S-transferase, and the accumulation of mutations in genes coding for the α or β chains of tubulin (Juliano, R.L. et al., Biochim. Biophys. Acta 455 (1976) 152-162; Beck, W.T. et al. Cancer Res. 39 (1979) 2070-2076.; Cole, S.P. et al., Science 258 (1992) 1650-1654; Fry, A.M. et al., Cancer Res. 51 (1991) 6592-6595; Giaccone, G. et al. Cancer Res. 52 (1992) 1666-1674; Balcer-Kubiczek, E.K. et al. Radiat. Res. 142 (1995) 256-262; Aas, T. et al., Nat. Med. 2 (1996) 811-814; Batist, G. et al., J. Biol. Chem. 261 (1986) 15544-15549; Batist, G. et al., Biochem. Pharmacol. 35 (1986) 2257-2259; Harris, A.L. et al., Acta Oncol. 31 (1992) 205-213; Cabral, F. et al., Proc. Natl. Acad. Sci. U.S.A 78 (1981) 4388-4391; Schibler, M.J. et al., J. Cell Biol. 113 (1991) 605-614).

Recently, genome profiling approaches have provided significant insight into the genes and mechanisms associated with the acquisition of drug resistance in breast tumour cells. (Parissenti, A.M. et al., Anticancer Drugs 18 (2007) 499-523; Villeneuve, D.J. et al., Breast Cancer Res. Treat. 96 (2006) 17-39).

The presence of multiple and varied mechanisms of intrinsic or acquired drug resistance makes it very difficult to identify which patients will respond to a given chemotherapy regimen and whether this response will be sustained throughout treatment. In patients, a sensitive tumour may regress or shrink during chemotherapy, and continue to regress following chemotherapy. In other patients, a resistant tumour can be unresponsive to chemotherapy both mid- and post-treatment. Finally, a tumour may regress during chemotherapy in some patients, but return to its original state (or continue to grow) after chemotherapy is completed.

It would be highly beneficial to be able to determine the level of tumour responsiveness to a given chemotherapeutic drug or agent before administration, or early after drug administration. For example, only 33% and 35.4% of breast cancer patients respond to paclitaxel and docetaxel after anthracycline-based chemotherapy, respectively (Seidman, A.D. et al., J. Clin. Oncol. 13 (1995) 1152-1159; Ando, M. et al., J. Clin. Oncol. 19 (2001) 336-342). However, biomarkers capable of distinguishing between chemotherapy-sensitive and chemotherapy-resistant tumours in cancer patients have yet to be identified. Thus, for cancer patients receiving chemotherapy regimens involving cytotoxic agents, there is no current method to determine whether a tumour is responding to chemotherapy mid-treatment or whether the viability of tumour(s) has been eradicated post-treatment. Consequently, cancer patients experience the serious negative side effects from taking cytotoxic drugs, without knowing whether their tumours are, in fact, responding to these agents.

Accordingly, there is a need for a method of quickly and accurately assessing the level of responsiveness of tumours to particular chemotherapy drugs (and combinations thereof), in order to tailor a specific regimen best suited to a patient's needs. There is a further need for indicators of sensitivity or resistance to chemotherapy drugs.

Balatsos et al (2001; J.Exp. Clin. Cancer Res. vol. 20 p63-69) discloses that *in vitro* treatment of lymphoma cells with interferon and 5-fluorouracil results in cell apoptosis. Ogston et al. (2003; The Breast vol. 12 p320-327) discloses a method of determining response of breast cancer to chemotherapy by measuring tumour cellularity before and after treatment. Carey et al. (1999; J. Clin. Oncol vol. 17 p 3075-3081) discloses that determining RNA stability is a way of measuring tumour cellularity.

### Summary of the Invention

In accordance the invention, there is provided a method of determining tumour responsiveness to a chemotherapeutic agent in a sample comprising tumour cell RNA, obtained from a patient with a cancerous tumour after the patient has received one or more doses of the chemotherapeutic agent, comprising determining the RNA integrity assessed by degradation of the sample and comparing the RNA integrity of the sample to a time point before administration of the one or more doses of the chemotherapeutic agent;
wherein a decrease in RNA integrity over time is indicative that the tumour is responsive to the chemotherapeutic agent and a high RNA integrity of the sample, wherein the extracted RNA exhibits little to no degradation, is indicative that the tumour is resistant to the chemotherapeutic agent.

In an embodiment of the invention, the RNA integrity is determined as a ratio of 28S and 18S rRNA intensity values, wherein said ratio is obtained by gel electrophoresis of the extracted RNA, ethidium bromide staining of said gel, and calculation of said ratio of intensities of 28S and 18S rRNA visualized under ultraviolet light.

In another embodiment of the invention, the RNA integrity is determined by capillary electrophoresis of the extracted RNA and quantification of the various RNAs separated in the electrophoresis. Preferably, the RNA integrity is quantified as an RNA integrity number (RIN), wherein the RIN is calculated by an algorithmic assessment of the amounts of various RNAs present within the extracted RNA.

More than one chemotherapeutic agent may be administered to the patient. The chemotherapeutic agent can be selected from the group consisting of anthracycline and taxane chemotherapeutic agents. Preferably, the chemotherapeutic agents comprise an anthracycline and a taxane. In an embodiment of the invention, epirubicin and docetaxel are used in the chemotherapy regimen.

In yet another preferred embodiment, the RNA is extracted from one or more core biopsies of a tumour of the patient. Preferably, the core biopsy is obtained by image-guided means such as computed tomography (CT), x-ray, ultrasound, and magnetic resonance imaging (MRI). The RNA quality is then determined from the one or more core biopsies of said tumour.

In another embodiment of the invention, the magnitude of reduction in the RNA integrity is proportionate to tumour reponsiveness, wherein tumour responsiveness may be assessed by a corresponding decrease in tumour extent and/or cellularity, and clinical response of the patient.

In another embodiment of the invention, a patient with a post-treatment RIN of 3 or less is identified as being responsive to the chemotherapeutic agent(s), and a patient with a post-treatment RIN of 3 or more is identified as being non-responsive to the chemotherapeutic agent(s).

An advantage of the present invention is that tumour RNA integrity, quantified as an RNA integrity number (RIN), is an easily accessed biomarker of tumour responsiveness to a particular chemotherapy regimen involving one or more chemotherapeutic agents.

Presently known methods of determining tumour responsiveness, which generally require the visual interpretation of photomicrographs of fixed and stained sections of core biopsies by a human operator such as a pathologist. Such methods are dependent on the subjective interpretation by the operator, which may vary from one person to the next. Such methods are also prone to human error. The assessment of tumour RIN provides a significant advantage over presently known methods of assessing tumour responsiveness to a given chemotherapy regiment, as the tumour RIN is a quantitative biomarker of tumour responsiveness that is both accurate and reproducible.

Another advantage of the present invention is that assessment of tumour RIN can be carried out by automated means. The automated means can involve high-throughput screening, which allows for rapid assessment of tumour RIN. The rapid assessment of tumour RIN thus allows rapid and accurate assessment of the level of responsiveness of a patient's tumour to a given chemotherapy regimen.

Another advantage of the present invention is that the RIN value of tumour cells may be correlated with the dosage level of the chemotherapeutic agent, thus allowing tailoring of a chemotherapy regimen to a patient's needs, or level of responsiveness.

Other and further advantages and features of the invention will be apparent to those skilled in the art from the following detailed description of an embodiment thereof, taken in conjunction with the accompanying drawings.

### Brief Description of the Drawings

The present invention will be further understood from the following detailed description of an embodiment of the invention, with reference to the drawings in which:
Figure 1 is a series of representative electropherograms of tumour RNA preparations after capillary electrophoresis, demonstrating the relationship between tumour RNA integrity (RIN) values and RNA quality;
Figure 2 is a graph of tumour RNA integrity (RIN) values by dose level for 50 MA.22 patients pre-, mid-, and post-treatment with epirubicin/docetaxel chemotherapy;
Figure 3 is a series of electropherograms showing typical patterns of change in tumour RNA profiles for MA.22 patients pre-, mid-, and post-treatment with epirubicin/docetaxel chemotherapy;
Figure 4 is a series of histograms showing typical patterns of change in tumour RIN values for MA.22 patients pre-, mid-, and post-treatment with epirubicin/docetaxel chemotherapy;
Figure 5 depicts photomicrographs of haematoxylin/eosin-stained sections of image-guided core biopsies of representative MA.22 patients pre-, mid-, and post-treatment with epirubicin/docetaxel chemotherapy; and
Figure 6 depicts the relationship between pathologic complete response (pCR) (dashed lines) and maximum tumour RIN values for 50 MA.22 patients at various drug dose levels, pre-, mid-, and post-treatment with epirubicin/docetaxel chemotherapy.

### Detailed Description of Embodiments

Ribonucleic acids (RNAs) play a number of essential roles in the translation of genetic information into functional proteins within eucharyotic cells. mRNAs are processed transcripts of genes, which bind to ribosomes for translation into specific proteins. Other RNAs form vital portions of ribosomes (rRNAs), or act as carriers for amino acids in protein synthesis (tRNAs).

The levels of cellular RNA are precisely regulated, maintaining a balance between transcription and RNA degradation pathways. There is increasing evidence that surveillance systems are present in cells to monitor RNA quality within the cell. These surveillance systems are coupled to RNA degradation pathways to rid cells of a variety of defective RNAs (Houseley, J. et al., Nat. Rev. Mol. Cell Biol. 7 (2006) 529-539; Parker, R. et al., Nat. Struct. Mol. Biol. 11 (2004) 121-127). Defective RNAs include inappropriately processed primary transcripts and mRNAs lacking translational stop codons, containing premature termination codons, or containing nonsense codons. In addition, there is evidence that RNA degradation is a hallmark of apoptosis (programmed cell death). Apoptosis-inducing agents have been shown to induce RNA degradation in cells (King, K.L. et al, Cell Death. Differ. 7 (2000) 994-1001; Bakhanashvili, M. et al., J. Mol. Med. (2007)). In particular, chemotherapy agents, particularly those generating reactive oxygen species, may induce sufficient damage to DNA and/or RNA, such that a variety of defective RNAs are produced and the above-noted RNA degradation pathways are activated.

Given the critical role which the various cellular RNAs play in cell function, the types of RNA and their intracellular concentrations can provide a significant amount of information on cellular activity, such as gene expression and protein production. Thus, it is desirable to extract cellular RNA in order to obtain a "snapshot" of what is happening within the cell at a given point in time. The extracted RNA can then be used to clone cDNAs into expression vectors, to identify and quantify mRNA transcripts by reverse transcription polymerase chain reaction (RT-PCR), and gene expression profiling by high-throughput RT-PCR or microarray studies. However, since RNA is susceptible to extensive degradation by RNAse enzymes which are ubiquitous in the environment, an assessment of RNA quality or integrity is essential before performing the above applications. The RNA "quality" or "integrity" (used interchangeably throughout) thus refers to the state of the RNA following extraction from the cell. High RNA quality is taken as meaning little to no degradation of the RNA following extraction, whereas low RNA quality means the extracted RNA exhibits a significant to total degradation.

In the past, RNA quality or integrity has been evaluated by visualization of RNA bands under ultraviolet light after gel electrophoresis and staining of the gel with ethidium bromide. Typically, the intensity values for the 28S and 18S rRNA bands are determined by film densitometry and a 28S/18S rRNA ratio computed. RNA is considered of high quality if the 28S/18S rRNA ratio is about 2.0 or higher. However, since the above approach relies on the interpretation of the gel and/or film densitometry by a human operator, it is subjective and the results are difficult to reproduce between different operators. In addition, large quantities of RNA are also required for this approach, making it difficult to obtain enough RNA for an analysis.

Recently, microcapillary electrophoresis has been used increasingly to assess RNA integrity, particularly since only nanogram quantities of RNA are required. One such platform, the Agilent® 2100 Bioanalyzer (Agilent Technologies, Inc., U.S.A.) uses microfluidics technology to carry out electrophoretic separations of RNAs in an automated, reproducible manner (Mueller, O. et al., Electrophoresis 21 (2000) 128-134). The Agilent® 2100 Bioanalyzer is now used in many laboratories for the assessment of RNA quality, particularly following the development of software for the Agilent® Bioanalyzer that calculates an RNA integrity number (RIN) for each sample after capillary electrophoresis. (Schroeder, A. et al., BMC. Mol. Biol. 7 (2006) 3; Imbeaud, S. et al. Nucl. Acids Res. (2005), 33, 6, e56, 1-12). This software incorporates an algorithm which quantifies the amounts of multiple RNAs in the electropherogram of a given RNA sample and assigns a RIN value based on this assessment. Recent studies suggest that the RIN is superior to the 28S/18S rRNA ratio for reliably measuring RNA quality. (Schroeder, A. et al., BMC. Mol. Biol. 7 (2006) 3; Weis, S. et al., J. Neurosci. Methods 165 (2007) 198-209; Strand, C. et al., BMC. Mol. Biol. 8 (2007) 38). The RIN is emerging as the best method for RNA quality assessment in mammalian cell lines and tissues, including tumours of the breast and other organs. (Fleige, S. et al., Biotechnol. Lett. 28 (2006) 1601-1613; Strand, C. et al., BMC. Mol. Biol. 8 (2007) 38).

It has now been discovered that RNA quality or integrity, as measured by tumour RNA quality, particularly as measured with the RIN value, can be used as a direct measure of cell viability.

A set of tumour biopsies were taken from 50 patients with breast cancer, who were undergoing an epirubicin/docetaxel chemotherapy regimen with pegflgrastim support (see Example 2). Two tumour biopsies were taken from each patient at three time points, before (pre-), during (mid-) and after (post-) chemotherapy treatment, to form two sets of biopsies for each patient (each set composed of a pre-, a mid- and a post-treatment biopsy). One set of biopsies was analyzed for the RNA quality (i.e. determination of RIN value) and the other set of biopsies was subjected to immunohistochemical analysis to determine levels of specific tumour marker proteins known to be important for breast cancer prognosis, percentage tumour cellularity and photomicrographs. Tumour RIN was then compared to the observed changes in tumour marker proteins (Example 2(d)), tumour cellularity (Example 2(f)) and photomicrographs (Example 2(g)) that occurred during the course of the chemotherapy treatment, and analyzed for statistically significant correlation between tumour RIN and the observed changes. Finally, tumour RIN was compared with the observed clinical response of the patient.

Dramatic reductions in RNA integrity of tumour cells were observed to occur in drug-sensitive tumours post-chemotherapy, while drug-resistant tumours were observed to retain high RNA integrity, resulting in disease progression and poor patient prognosis. As noted in Example 2(b), a high drug dose level was strongly associated with a large negative change in tumour RIN during the course of treatment. Also, a low drug dose level correlated with few or small reductions in tumour RIN. This suggested that a reduction in tumour RIN was directly related to chemotherapy drug response in these patients. A strong positive correlation as found to between tumour extent (cellularity) and tumour RIN values measure post-treatment (see Example 2(e)). That is, a decrease in tumour extent was proportionate to the decrease in tumour RIN. Finally, tumour RIN measured post-treatment was found to be an accurate predictor of tumour response to chemotherapy and observed clinical response (see Example 2(f),(g)).

The response of tumours to a specific chemotherapy regimen in cancer patients can thus be effectively determined by monitoring the ability of the regimen to induce a reduction in tumour RNA quality (integrity).

Tumour RNA integrity can be measured by capillary electrophoresis, followed by the assignment of a RIN value. Chemotherapy-induced reductions in tumour RIN values would be indicative of responsive tumours, while little change in tumour RIN values would suggest that the tumour is resistant to the selected regimen.

In order to determine a cancer patient's responsiveness to a chemotherapy regimen, RNA is extracted from the patient's tumour(s) at least two different time points during the administration of a chemotherapy regimen. Preferably, RNA is extracted from the tumour before the administration of a chemotherapy regimen, and during and/or after completion of the regimen.

The chemotherapy regimen can consist of one chemotherapy agent or a combination of two or more chemotherapy agents, and the doses of each agent may be varied with time.

To improve reproducibility and accuracy, the tumour cells are preferably collected in one or more image-guided biopsies. To further improve reproducibility and accuracy, three or more image-guided biopsies are collected from the tumour. An image-guided biopsy is obtained with image-guided means such as computed tomography (CT), x-ray, ultrasound, and magnetic resonance imaging (MRI).

The quality of the extracted RNA is then determined. This can be done by traditional means such as obtaining the 28S/18S rRNA ratio as noted above. However, RNA quality is preferably determined by capillary electrophoresis of the extracted RNA and quantification of the RNAs in the resultant electropherogram. An automated analytical system, such as the Agilent® 2100 Bioanalyzer (Agilent Technologies, Inc., U.S.A.), is preferred for carrying out this determination, as such a system can assess the electropherogram and quantify the quality of a given RNA sample as an RNA integrity number (RIN). The Agilent® 2100 Bioanalyzer calculates the RIN using an algorithm which is incorporated in the software associated with the Bioanalyzer (Schroeder, A. et al., BMC. Mol. Biol. 7 (2006) 3; Imbeaud, S. et al. Nucl. Acids Res. (2005), 33, 6, e56, 1-12). The resultant RIN values are reproducible from one operator to the next, and can be processed digitally. Moreover, an automated analytical system such as the Agilent® 2100 Bioanalyzer allows rapid, high-throughput analyses of RNA samples. Thus, a patient's responsiveness to a given chemotherapy regimen can be determined during or after a chemotherapy regimen, and the regimen may be changed after no tumour response is detected. This is of great benefit as it identifies patients that have not responded to the chemotherapy regimen and would likely be at high risk of disease progression.

The RIN value of the tumour cells collected before administration of the chemotherapy regimen is then compared with the one or more RIN values of tumour cells collected after commencement of the regimen, i.e. during and/or after completion of the chemotherapy regimen. If a patient exhibits no change in tumour RNA integrity during treatment (response pattern A as noted in Example 2), then the patient's tumour would be considered resistant to the chemotherapy regimen being used. The patient would be considered non-responsive to the chemotherapy regiment, i.e. at high risk of tumour progression and prognosis would be considered poor. Alternative chemotherapy regimens or treatment protocols can then be considered, such as a change in dosage level and/or a change in the type of chemotherapy agent(s) being administered. The method outlined herein can then be repeated to determine responsiveness to the new regimen, thus allowing tailoring of a chemotherapy regimen according to the patient's response.

If a patient exhibits a dramatic reduction in tumour RNA integrity (>50%) both mid- and post-treatment (response pattern C as noted in Example 2), then the patient would be considered to have responded to chemotherapy and would be at lower risk of tumour progression. The patient's prognosis would be considered good. Tumour RIN values near zero would be highly indicative of response to chemotherapy and low risk of tumour progression.

If a patient exhibits a dramatic reduction in tumour RNA integrity post-treatment only (response pattern B as noted in Example 2), then the patient would be considered to have responded to chemotherapy and be at lower risk of tumour progression. The patient's prognosis would be considered good.

If a patient exhibits a dramatic change in tumour RNA integrity mid-treatment only, then she likely has responded to therapy and would be at a lower risk of disease recurrence. This is regardless of a return to high "tumour" RNA integrity post-treatment, since the high quality RNA post-treatment may stem from normal tissue that has infiltrated the lesion. However, it is possible that the tumour has recurred post-treatment.

Further details of the preferred embodiments of the invention are illustrated in the following Examples which are understood to be non-limiting with respect to the appended claims.

### Example 1: Materials and Methods

### (a) Total RNA isolation from breast tissue core biopsies.

RNA was isolated from patient tumour core biopsies using QIAGEN® RNAeasy® mini kits (Qiagen GmbH, Germany). The RNA isolation protocol was slightly modified from the protocol published by Qiagen GmbH (freely available from Qiagen GmbH, Germany; also available at
http://www1.qiagen.com/literature/handbooks/literature.aspx?id=1000291).

Image-guided needle core biopsies of the patients tumour were taken from the patient, immediately touch prepared to a glass slide for determination of tumour cellularity, and the core biopsy immediately flash frozen on dry ice for future analysis. The frozen core biopsies were immediately dropped in 0.5 ml of RLT buffer containing β-ME (10 µl into 1 ml) in a Eppendorf tube. The biopsies in RLT buffer were homogenized with a Coreless™ motor homogenizer for 5 min (Kontes Glass Company, U.S.A., Cat#:749540-0000).

The lysate was then passaged at least 5 times through a 20-gauge needle (0.9 mm diameter) fitted to an RNase-free syringe. The sample was then centrifuged at high speed in a refrigerated microfuge at 4°C for 3 min., with transfer of the supernatant to a new tube.

One volume (500 µl) of 70% ethanol was then added to the supernatant and the sample mixed well by repeated pipetting. If some lysate was lost during homogenization, then the volume of ethanol was adjusted accordingly. Visible precipitates formed after the addition of ethanol in some samples did not affect the RNA isolation procedure.

A maximum of 700 µl of the sample, including any precipitate, were added to a RNeasy^{®} mini column and placed in a 2 ml collection tube. The column was centrifuged for 15 s at ∼8000 x g (∼10,000 rpm) and the flow-through discarded. The remainder of the sample was then added to the column and the column centrifuged again.

Seven hundred µl of Buffer RW1 was then added to the RNeasy® column and the column centrifuged for 15 s at ∼8000 x g (∼10,000 rpm) to wash the column. The flow-through was discarded.

The RNeasy® column was transferred into a new 2 ml collection tube and 500 µl of Buffer RPE was applied to the column. The column was then centrifuged for 15 s at ∼8000 x g (∼10,000 rpm) to wash the column. The flow through was discarded.

The RNeasy® column was transferred to a new 2 ml collection tube, discarding the old collection tube and flow-through. The column was then centrifuged again in a microcentrifuge at full speed for 1 min., discarding the collection tube and flow-through once again.

To elute the bound RNA, the RNeasy® column was transferred to a new 1.5 ml collection tube. Thirty µl of RNase-free water was applied directly to the column and the column centrifuged for 1 min. at ∼8000 x g (∼10,000 rpm).

To obtain a higher total RNA concentration for the sample, a second elution step was performed using the eluate from step 8.

The concentration and quality of RNA was then checked using an Agilent® 2100 Bioanalyzer and associated software.

### (b) Measurement of RNA quantity and RNA integrity

The total RNA sample from the tumour core biopsy was applied to RNA 6000 Nano Lapchips™ (Agilent Technologies, Inc.) and subjected to capillary electrophoresis using an Agilent® 2100 Bioanalyzer. The protocol for the Agilent® 2100 Bioanalyzer (Agilent Technologies, Inc.) was followed (Agilent® 2100 Bioanalyzer User's Guide, ed. Nov. 2003, Manual Part No. G2946-90000, Agilent Technologies, Inc., available at http://www.chem.agilent.com/temp/rad4DEAE/00000725.PDF).

The amount of RNA in the sample and the quality of the RNA (RNA integrity) was determined using the RIN algorithm disclosed by Schroeder et al. (Schroeder, A. et al. "The RIN: an RNA integrity number for assigning integrity values to RNA measurements", *BMC. Mol. Biol.* 7 (2006) 3.), which is incorporated in the computer software associated with the Agilent® 2100 Bioanalyzer (the software and accompanying manual are freely available from Agilent Technologies Inc., and also at http://www.chem.agilent.com/scripts/generic.asp?lpage=52241&indcol=N&prodcol= Y)

### Example 2: The RNA Integrity Number (RIN) and Measurement of Tumour RNA Quality in Breast Cancer Patients

### (a) Tumour biopsy samples from breast cancer patients in chemotherapy clinical trial

To test whether treatment of breast cancer patients with chemotherapy agents results in tumour RNA degradation, six image-guided core biopsies of tumours were taken from 50 patients with locally advanced or inflammatory breast cancer pre-, mid-, and post-treatment with epirubicin/docetaxel chemotherapy. Patients were from a national clinical trial hosted by the National Cancer Institute of Canada Clinical Trials Group (referred to as group "MA.22") and were treated with increasing dose levels of both epirubicin and docetaxel, with pegfilgrastim support to reduce neutropenia associated with this therapy. Chemotherapy was administered in a standard dosing regimen (Arm A) every 3 weeks, and the dose levels used in this study are depicted in Table 1. The maximum tolerated dose for this regimen was dose level 6, i.e. 105 mg/m² epirubicin and 75 mg/m² docetaxel (see Schedule A, Table 1).

**Table 1: Dose levels of epirubicin, docetaxel, and pegfilgrastim administered to patients with locally advanced/inflammatory breast cancer using either a 3-weekly (schedule A) or 2-weekly (schedule B) regimen in association with a clinical trial (MA.22) by the National Cancer Institute of Canada.**

| **Dose Level** | **Epirubicin (mg/m² IV)** | **Docetaxel (mg/m² IV)** | **Pegfilgrastim (mg per cycle, day 2)** |
|---|---|---|---|
| 1-Schedule B | 50 | 50 | 6 |
| 2-Schedule B | 60 | 60 | 6 |
| 3-Schedule B | 70 | 70 | 6 |
| 4-Schedule A | 75 | 75 | 6 |
| 5-Schedule A | 90 | 75 | 6 |
| 6-Schedule A | 105 | 75 | 6 |
| 7-Schedule A | 120 | 75 | 6 |

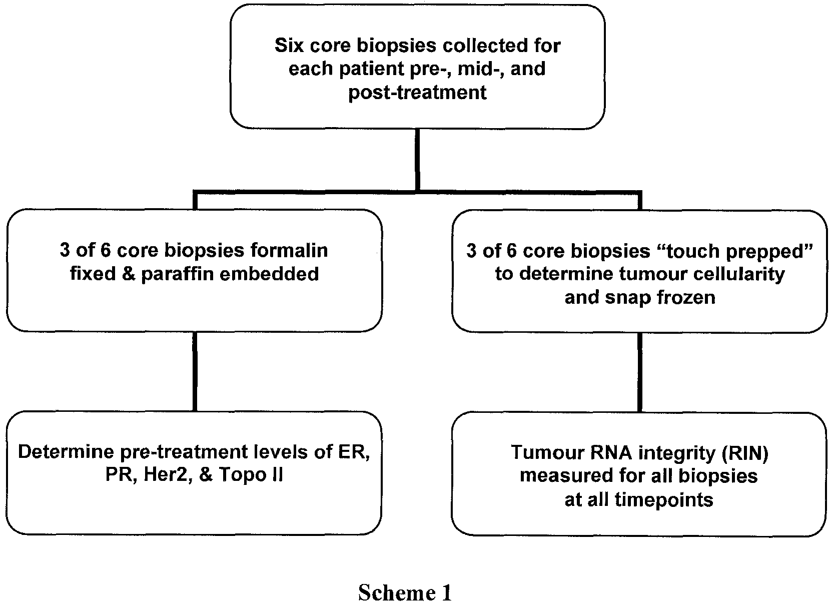

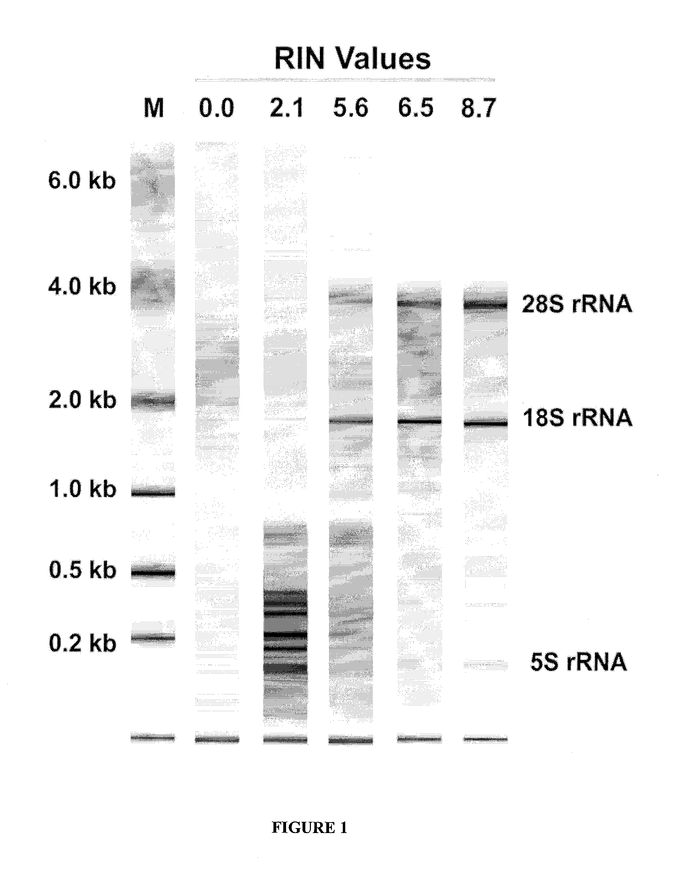

As noted in Scheme 1, three of the six biopsies taken from each patient were freshly frozen for RNA quality studies, while the remainder were fixed in formalin for assessment of levels of specific tumour markers, including the estrogen receptor ER (Novacastra® Clone 6G11, Leica Microsystems, Germany), the progesterone receptor PR (Novacastra® Clone 16, Leica Microsystems, Germany), topoisomerase II ("Topo II"; clone SWT3D1, Dako Denmark A/S) and human epidermal growth factor receptor 2 ("Her2"; Zymed® TAB250, Invitrogen Corp., U.S.A.). RNA was isolated from two of the freshly frozen core biopsies using RNeasy® Mini kit (Qiagen GmbH, Germany), after which the RNA quality of the sample was assessed by capillary electrophoresis using an Agilent® 2100 Bioanalyzer. The Bioanalyzer quantified the abundance of specific RNAs in the sample and assigned an RNA integrity number (RIN) to each sample. As shown in Figure 1, the magnitude of the RIN was observed to be a reliable measure of RNA integrity as visualized by inspection of electropherograms after capillary electrophoresis of tumour RNA preparations, i.e. the intensities of the 28S and 18S rRNA bands decreased noticeably with decreasing RIN value.

### (b) Association between Changes in RNA Integrity and Drug Dose Level

The mean core RIN values for RNA isolated from patient core biopsies were then assessed to determine if these values fell in response to epirubicin/docetaxel chemotherapy and whether there was a relationship between drug dose level and the magnitude of RIN reduction. While there was variation in pre-treatment mean tumour RIN values for patients, they were rarely below 5.0, with a mean value of 6.5 when data from all 50 patients was assessed (Table 2). The association between RIN and baseline drug dose was assessed using a 1-way ANOVA.

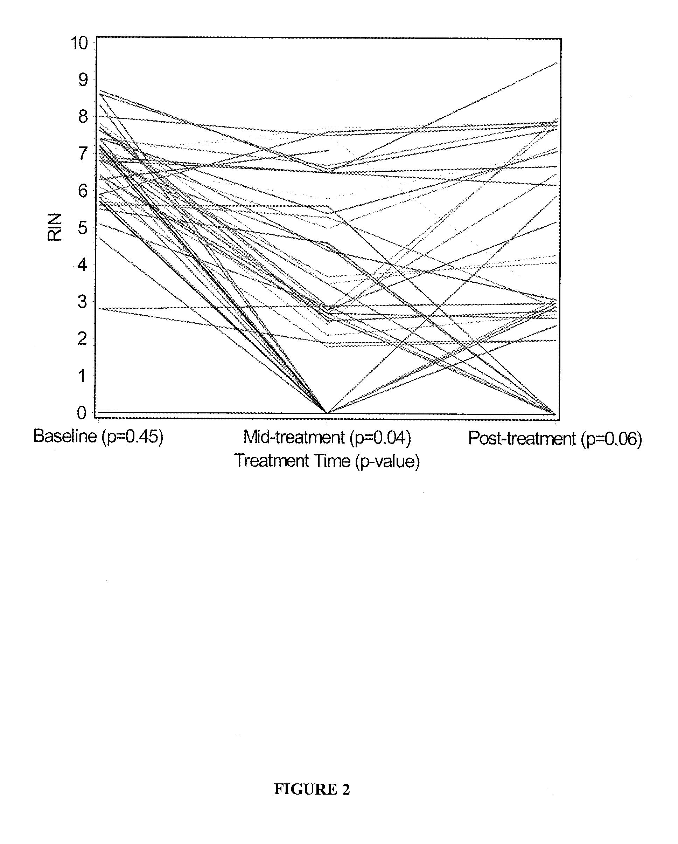

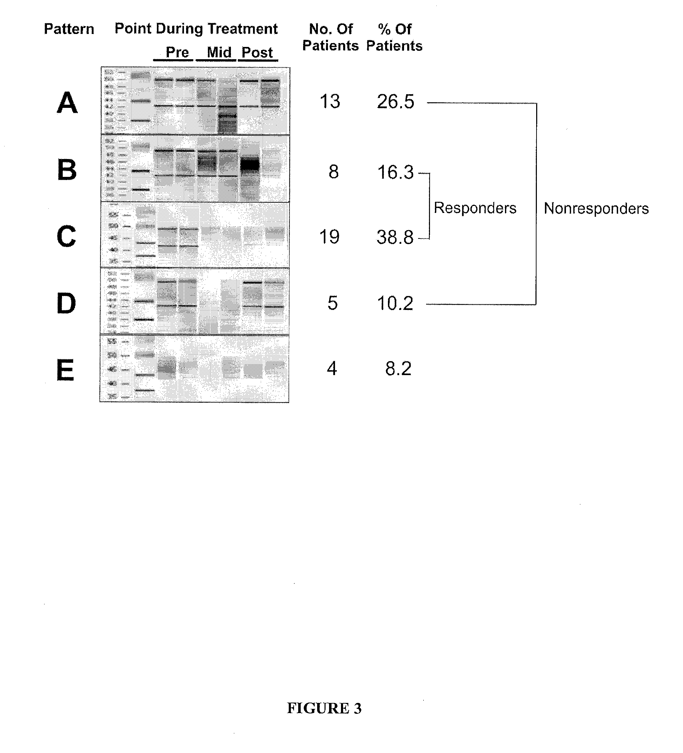

As expected, there was no association between the magnitude of the tumour RIN and drug dose level at baseline (p=0.45), given that patients had yet to receive chemotherapy. In contrast, RIN values were significantly and negatively correlated with drug dose level mid-treatment (p=0.04). Few or small reductions in the tumour RIN were observed for patients receiving low drug doses, while patients receiving high drug doses exhibited dramatic reductions in the tumour RIN (Figure 2). Despite the small number of patients (n=3), the effect of chemotherapy on tumour RIN values was particularly evident for tumours exposed to dose level 7, where mean RIN values were zero in mid- and post-treatment samples, but 5.0 in pre-treatment samples (Table 2). The mean mid-treatment RIN value across all doses was 3.8. The mean tumour RIN value post-treatment was 4.2, also suggesting a significant decrease in tumour RNA integrity after chemotherapy. A similar negative relationship between tumour RIN values and drug dose levels was observed post-treatment, but with borderline significance (p=0.06) (Figure 3). The borderline significance value post-treatment may be the result of two possible phenomena: the tumour cell population recovered once the chemotherapy drugs cleared the circulation (resulting in disease recurrence) or the tumour became infiltrated with normal tissues and/or cell types. In either instance, the tumour RIN values would be expected to increase, reducing the ability to detect chemotherapy-induced changes in tumour RNA quality.

**Table 2 - Tumour RIN values for patients in the MA.22 clinical trial pre-, mid-, and post-treatment with epirubicin/docetaxel chemotherapy at the dose levels depicted in Table 1. ¹If the number of patients (N) = 1, then the value of RIN for that patient is provided rather than an estimate of the mean. ²If N=2, then the range of RIN values is provided in place of the estimate of 95% confidence limit. ³Superscript 3 indicates truncation at zero.**

| **Treatment Time** | **Dose Level** | **N** | **Mean** | **(95% Confid. Limit)** |
|---|---|---|---|---|
| | | | | |
| **Baseline** | **All Patients** | **50** | **6.5** | **(6.1, 6.8)** |
| | | | | |
| | 1 | 3 | 6.9 | (6.7, 7.2) |
| | 2 | 2 | N/A | (6.4, 8.2) |
| | 3 | 1 | 7.2 | (N/A, N/A) |
| | 4 | 3 | 7.3 | (6.8, 7.7) |
| | 5 | 6 | 6.5 | (5.9,7.0) |
| | 6 | 32 | 6.3 | (5.8, 6.9) |
| | 7 | 3 | 5.2 | (0.0,7.7) |
| | | | | |
| **Mid-treatment** | **All Patients** | **50** | **3.8** | **(3.0, 4.5)** |
| | | | | |
| | 1 | 3 | 7.0 | (5.8,8.1) |
| | 2 | 2 | N/A | (1.0, 4.0) |
| | 3 | 1 | 2.4 | (N/A, N/A) |
| | 4 | 3 | 3.8 | (2.2, 5.0) |
| | 5 | 6 | 3.3 | (0.9, 4.6) |
| | 6 | 32 | 3.8 | (2.7, 4.6) |
| | 7 | 3 | 0.0³ | (0.0, 0.0)³ |
| | | | | |
| **Post-treatment** | **All Patients** | **49** | **4.2** | **(3.2,4.5)** |
| | | | | |
| | 1 | 3 | 6.3 | (2.9, 8.6) |
| | 2 | 2 | n/a | (1.5, 4.9) |
| | 3 | 1 | 8.0 | (N/A, N/A) |
| | 4 | 3 | 6.5 | (3.9, 8.4) |
| | 5 | 6 | 4.4 | (0.2, 6.4) |
| | 6 | 31 | 3.7 | (2.2, 4.7) |
| | 7 | 3 | 0.0³ | (0.0, 0.0)³ |

### (c) Patterns of Change in RNA Integrity During Treatment of Patients with Epirubicin/Docetaxel Chemotherapy

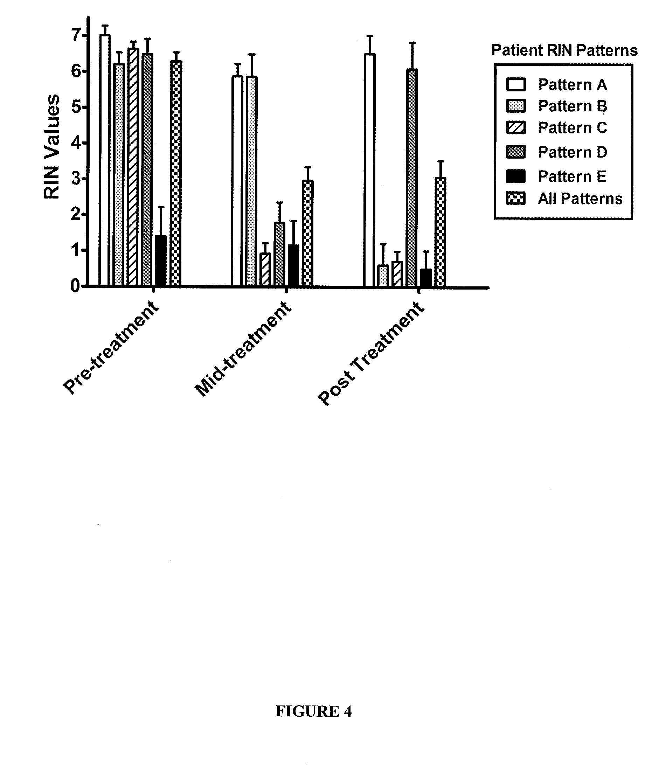

Figure 3 depicts the patterns of change in tumour RNA quality observed in the MA.22 patients during epirubicin/docetaxel chemotherapy as assessed by visual inspection of electropherograms after capillary electrophoresis. Patients exhibited no change in RNA quality (pattern A), a temporary reduction in RNA quality mid-treatment only (pattern D), a strong reduction in RNA quality post-treatment only (pattern B), or dramatic reductions in RNA quality mid- and post-treatment (pattern C). For a small number of patients, tumour RNA quality was poor at all timepoints (pattern E), the cause of which was unknown. These patterns were also reflected in the corresponding patient RIN values pre-, mid-, and post-chemotherapy (Figure 4). Patient tumours exhibiting patterns B and C were taken to be responders to chemotherapy (∼55% of patients), while patient tumours exhibiting patterns A and D were taken as nonresponders to treatment (∼37% of patients).

### (d) Relationship between Topoisomerase II Levels and Tumour RNA Integrity

Immunohistochemical approaches were then used to determine baseline levels of specific tumour marker proteins known to be important for breast cancer prognosis, and expression was rated as a percentage of positive stain against a known standard. Proteins assessed by immunohistochemistry included the estrogen receptor (ER), the progesterone receptor (PR), human epidermal growth factor receptor 2 (Her2) and topoisomerase II ("topo II"). Associations between pre-treatment levels of specific tumour markers and RIN values at various time points were then assessed by computing Spearman and Pearson correlation coefficients, with or without data high pre-treatment levels of topo II were significantly associated with high tumour RIN pre-treatment (Table 3; p values between 0.01 and 0.03). The association of high pre-treatment RIN values with high pre-treatment topo II levels suggested that cells with high topo II expression are highly viable, rapidly proliferating, and produce high quality RNA. This association was also evident post-treatment (see Table 3). Tumours with high RIN values (i.e. high quality RNA) post-treatment, were taken as representing either highly viable tumours not responding to chemotherapy or healthy normal tissue that had infiltrated the tumour. No association was observed between pre-treatment levels of Her2, ER or PR and RIN values pre- or post-treatment (data not shown).

**Table 3 - Relationship between tumour RNA integrity number (RIN) and topoisomerase II expression for MA.22 patients before chemotherapy (baseline), mid-treatment, and post-treatment. Per cent topoisomerase II expression was determined by a pathologist through immunohistochemistry experiments probing fixed sections of tumour core biopsies from the patients with a topoisomerase II α-specific antibody. Data was assessed using transformed or untransformed values to control for data variance. Both Pearson and Spearman correlation co-efficients were determined and p values indicating statistical significance are highlighted in bold.**

| **Factors** | **Pearson Correlation** | **P value** | **Spearman Correlation** | **P value** |
|---|---|---|---|---|
| **Maximum RIN and Topo II** | | | | |
| Transformed RIN, untransformed Topo2 | | | | |
| Baseline RIN | 0.39 | **0.01** | 0.30 | **0.03** |
| Mid-treatment RIN | 0.22 | 0.13 | 0.16 | 0.28 |
| Post-treatment RIN | 0.35 | **0.02** | 0.27 | 0.07 |
| Untransformed RIN, Topo2 | | | | |
| Baseline RIN | 0.34 | **0.02** | 0.30 | **0.03** |
| Mid-treatment RIN | 0.23 | 0.12 | 0.16 | 0.28 |
| Post-treatment RIN | 0.30 | **0.04** | 0.27 | 0.07 |

### (e) Relationship between Tumour RIN Values and Tumour Cellularity

One measure of drug response in patients involves assessing the magnitude of reduction in the number of tumour cells comprising a lesion(s) post-therapy. As summarized in Table 4 below, the changes in RIN values corresponded to various patterns of change in tumour cellularity values. Overall, patients exhibited significant responses to chemotherapy, given that the overall tumour extent or cellularity fell from 90.94% ± 2.17% to 50.69% ± 5.79% mid-treatment and 39.6% ± 5.78% post-treatment (see Table 4). Patients who exhibited the following response patterns were placed into Groups A to E as follows: (A) no change in tumour cellularity; (B) a strong reduction in tumour cellularity post-treatment only; (C) dramatic reductions in tumour cellularity mid- and post-treatment; and (D) a temporary reduction in tumour cellularity mid-treatment only. Patients for whom the data was incomplete were placed in Group E. When all patients were assessed simultaneously, statistically significant reductions in tumour cellularity were observed mid- and post-chemotherapy.

**Table 4. Variations in response of MA.22 patients to epirubicin/docetaxel chemotherapy, as measured by strong reductions in tumour cellularity mid- or post-treatment.**

| **Tumour Cellularity** | | | |
|---|---|---|---|
| **Patient ID Number** | **Pre-treatment** | **Mid-treatment** | **Post-treatment** |
| | | | |

| **Group A** - **Nonresponders to Epirubicin/Docetaxel Chemotherapy** | | | |
|---|---|---|---|
| CAMN004 | 100 | 100 | 100 |
| CAMN002 | 100 | 90 | 100 |
| CAMN006 | 100 | 90 | 100 |
| CAMN024 | 100 | 100 | 100 |
| CAMN028 | 100 | 100 | 90 |
| CAMN031R | 100 | 100 | 50 |
| CAMN043 | 100 | 100 | 100 |

| **Tumour Cellularity** | | | |
|---|---|---|---|
| **Patient ID Number** | **Pre-treatment** | **Mid-treatment** | **Post-treatment** |
| CAGS005 | 80 | 80 | 80 |
| CAMN007 | 100 | 50 | 70 |
| CAMN018 | 100 | 80 | 90 |
| CAMN029 | 100 | 90 | 100 |
| CAMN034 | 70 | 50 | 90 |
| CAMN036 | 100 | 90 | 50 |
| CAGS002 | 80 | 80 | 50 |
| CAMN013 | 60 | 60 | 70 |
| CAMN021 | 70 | 90 | 50 |
| MEAN (± S.E.) | 91.25 ± 3.52 | 84.38 ± 4.28 | 80.63 ± 5.20 |
| | | | |

| **Group B** - **Response to Epirubicin/Docetaxel Chemotherapy only Post-treatment** | | | |
|---|---|---|---|
| CAMIN035 | 80 | 80 | 10 |
| CAMN041 | 100 | 80 | 0 |
| CAMN037 | 100 | 80 | 30 |
| CAMN008 | 50 | 100 | 0 |
| CAMN009 | 90 | 70 | 10 |
| CAMN015 | 100 | 90 | 10 |
| CAMN017 | 100 | 90 | 0 |
| CAMN020 | 100 | 50 | 0 |
| CAMN026 | 90 | 50 | 30 |
| CAMN045 | 90 | 90 | 5 |
| MEAN (± S.E.) | 90.00 ± 4.94 | 78.00 ± 7.55 | 9.5 ± 3.69 |
| | | | |

| **Group C - Response to Epirubicin/Docetaxel Chemotherapy Mid- and Post-treatment** | | | |
|---|---|---|---|
| CAMN039 | 90 | 2 | 0 |
| CAMN027 | 100 | 0 | 0 |
| CAMN005 | 100 | 1 | 0 |
| CAGS004 | 90 | 0 | 0 |
| CAMN023 | 100 | 0 | 0 |
| CAMN030 | 100 | 30 | 0 |
| CAMN044 | 100 | 0 | 20 |
| CAMN011 | 100 | 0 | 0 |
| CAMN012 | 90 | 0 | 0 |
| CAMN016 | 100 | 0 | 0 |
| CAMN033 | 90 | 20 | 0 |
| CAMN038 | 100 | 0 | 0 |
| CAGS001 | 90 | 20 | 5 |
| MEAN (± S.E.) | 96.15 ± 1.40 | 5.62 ± 2.88 | 1.92 ± 1.55 |
| | | | |

| **Group D - Response to Epirubicin/Docetaxel Chemotherapy only Mid-treatment** | | | |
|---|---|---|---|
| CAMN003 | 40 | 10 | 50 |
| CAMN010 | 100 | 0 | 50 |
| CAMN001 | 60 | 20 | 50 |
| CAMN025 | 100 | 0 | 100 |

| **Tumour Cellularity** | | | |
|---|---|---|---|
| **Patient ID Number** | **Pre-treatment** | **Mid-treatment** | **Post-treatment** |
| CAMN040 | 50 | 1 | 70 |
| CAMN047 | 60 | 0 | 50 |
| MEAN (± S.E.) | 68.33 ± 10.47 | 5.17 ± 3.37 | 61.67 ± 8.33 |
| | | | |

| **Group E** - **Incomplete Data** | | | |
|---|---|---|---|
| CAMN042 | 100 | 100 | |
| CAMN032 | 100 | 100 | |
| CAMN014 | 100 | 50 | |
| CAMN019 | 100 | 0 | |
| CAMN022 | 100 | | |
| | | | |
| **MEAN OF ALL SAMPLES (± S.E.)** | **90.94 ± 2.17** | **50.69 ± 5.79** | **39.6 ± 5.78** |

Given the strong correspondence between the patterns of change in RIN values and tumour cellularity values during treatment, the tumour RIN values were assessed to see if the changes in RIN were an accurate reflection of treatment response in patients (as measured by changes in tumour extent or cellularity). The relationship between the percentage of tumour cells in core biopsies and the maximum RIN value for core biopsies pre-, mid-, and post-treatment was analyzed and summarized in Table 5.

**Table 5 - Relationship between tumour RNA integrity number (RIN) and tumour extent for MA.22 patients before chemotherapy (baseline), mid-treatment, and post-treatment. Tumour extent (per cent tumour cellularity) was determined by a pathologist through microscopic visualization of fixed sections of tumour core biopsies from the patients stained with haematoxylin/eosin. Data was assessed using transformed or untransformed values to control for data variance. Both Pearson and Spearman correlation co-efficients were determined and p values indicating statistical significance are highlighted in bold.**

| **Factors** | **Pearson** | **P value** | **Spearman** | **P value** |
|---|---|---|---|---|
| **Maximum RIN** | **Correlation** | | **Correlation** | |
| Transformed RIN, untransformed Tumour Extent | | | | |
| | | | | |
| Baseline RIN | 0.15 | 0.29 | 0.09 | 0.55 |
| Mid-treatment RIN | 0.06 | 0.69 | -0.01 | 0.95 |
| Post-treatment RIN | 0.52 | **0.0003** | 0.42 | **0.004** |
| | | | | |

| Untransformed RIN, Tumour Extent | | | | |
|---|---|---|---|---|
| | | | | |
| Baseline RIN | 0.20 | 0.17 | 0.09 | 0.55 |
| Mid-treatment RIN | 0.01 | 0.93 | -0.01 | 0.95 |
| Post-treatment RIN | 0.49 | **0.001** | 0.42 | **0.004** |

As shown in Table 5, there was a very strong positive relationship between tumour extent values and RIN values post-treatment (p values ranged from 0.0003 and 0.004, depending upon whether Spearman or Pearson coefficients were computed and whether the data was transformed to improve symmetry and stabilize variances). The strong correlation between RIN values and patient response to chemotherapy (as measured by tumour cellularity levels) was not observed mid-treatment, possibly because the effects of the chemotherapy agents on tumour RIN had not been fully realized at the mid-point of treatment.

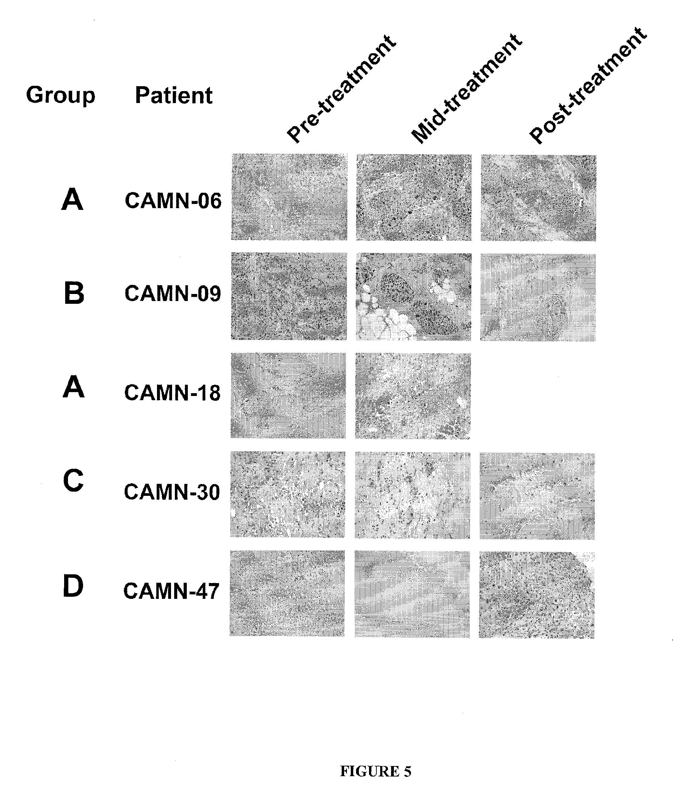

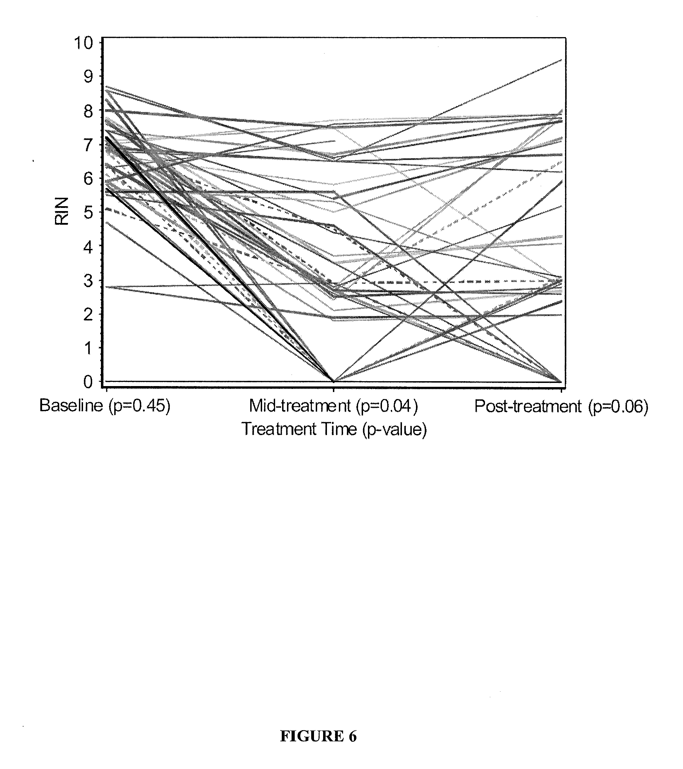

Photomicrographs of sections of tumours pre-, mid-, and post-treatment from patients from each of Groups A (CAMN-006; CAMN-018), B (CAMN-009), C (CAMN-047) and D (CAMN-030) are shown in Figure 6. As can be seen in Figure 5, the observed level of tumour cellularity corresponded to the changes observed in the tumour RIN.

### (f) Ability of Tumour RIN Values to Identify Tumour Response to Chemotherapy

The collected RIN values were examined to see whether post-treatment maximum tumour RIN values of ≤ 3.1 could accurately identify patients whose tumours were responding to chemotherapy (as measured by post-treatment tumour cellularities to ≤ 10%). A RIN value of 3.1 was selected, as it represented half of the mean RIN value of all pre-treatment tumour core biopsies except those in Group E (see Table 4, above). As shown in Table 6 (see below), patients that had a mean post-treatment tumour RIN value of 3.1 or less also had a post-treatment tumour cellularity of <10% in 19 of 20 patients (95% agreement). Moreover, 16 of 25 patients that had post-treatment tumour RIN values of >3.0 has post-treatment tumour cellularity values of ≥10% (64% agreement). Thus, given specific cut-off values, there was a good correlation between post-treatment RIN values and post-treatment tumour cellularity values, in particular for responders. However, discrepancies in this relationship occurred in four instances where tumour cellularity levels were observed to be high (≥50%) and RIN values were zero. Because breast tumours are known to be heterogeneous, some regions of the tumour may have high tumour cellularity, while other regions do not, thus resulting in discordance between tumour RIN and tumour cellularity. Alternatively, this may be due to tumour cells retaining their cellular morphology but being nonviable with completely hydrolyzed RNA. Therefore, tumour RIN measurements may be superior to tumour cellularity measurements in determining tumour response to chemotherapy agents, since tumour RIN serves as a functional biomarker.

**Table 6 - Ability of maximum tumour RIN to predict response to epirubicin/docetaxel chemotherapy in MA.22 patients (as determined by a reduction in tumour cellularity to ≤10%). Maximum RIN is the highest of two RIN values obtained from RNA isolated from two independent tumour core biopsies from each patient.**

| **Group A** | | | | | | | |
|---|---|---|---|---|---|---|---|
| | Cycle 0 | | | Cycle 3/4 | | Cycle 6/8 | |
| Patients (13) | Max RIN | % Tumour Cellularity | Her2 | Max RIN | % Tumour Cellularity | Max RIN | % Tumour Cellularity |
| CAMN001 | 7.1 | 60 | 0 | 5 | 20 | 7.2^{b} | 50^{b} |
| CAMN002 | 7 | 100 | 0 | 3.7 | 90 | 4.1^{b} | 100^{b} |
| CAMN006 | 7.4 | 100 | 80 | 6.7 | 90 | 7.9^{b} | 100^{b} |
| CAMN010 | 7.4 | 100 | 80 | 5.4 | 0 | 7.1^{b} | 50^{b} |
| CAMN024 | 8.7 | 100 | 80 | 6.5 | 100 | 9.5^{b} | 100^{b} |
| CAMN025 | 5.9 | 100 | 0 | 7.6 | 0 | 7.9^{b} | 100^{b} |
| CAMN028 | 6.8 | 100 | 0 | 6.5 | 100 | 6.2^{b} | 90^{b} |
| CAMN031R | 6.9 | 100 | 0 | 6.5 | 100 | 6.7^{b} | 50^{b} |
| CAMN037 | 8.6 | 100 | 0 | 6.6 | 80 | 7.7^{b} | 30^{b} |
| CAMN040 | 6.7 | 50 | 0 | 5.8 | 1 | 7.1^{b} | 70^{b} |
| CAMN043 | 7 | 100 | 0 | 7.7 | 100 | 7.9^{b} | 100^{b} |
| CAMN044 | 7.8 | 100 | 0 | 3.5 | 0 | 4.3^{b} | 20^{b} |
| CAGS005 | 8 | 80 | 0 | 7.5 | 80 | 7.8^{b} | 80^{b} |
| **Mean RIN±SE** | **7.33±0.22** | | | **6.08±0.38** | | **7.03±0.41** | |
| | | | | | | | |

| **Group B** | | | | | | | |
|---|---|---|---|---|---|---|---|
| | Cycle 0 | | | Cycle 3/4 | | Cycle 6/8 | |
| Patients (8) | Max RIN | % Tumour Cellularity | Her2 | Max RIN | % Tumour Cellularity | Max RIN | % Tumour Cellularity |
| CAMN007 | 5.7 | 100 | 0 | 5.3 | 50 | 2.9^{c} | 70^{c} |
| CAMN023 | 5.6 | 100 | 0 | 5.6 | 0 | 0^{a} | 0^{a} |
| CAMN030 | 6.8 | 100 | 0 | 4.5 | 30 | 0^{a} | 0^{a} |
| CAMN035 | 7.0 | 80 | 0 | 3.5 | 80 | 0^{a} | 10^{a} |
| CAMN041 | 7.1 | 100 | 0 | 7.5 | 80 | 3^{a} | 0^{a} |
| CAMN046 | 5.2 | 0 | 0 | 5.5 | 100 | 3.1 | |
| CAGS003 | 5.3 | 40 | 0 | 4.6 | | 0 | |
| CAGS004 | 7.6 | 90 | 100 | 4.4 | 0 | 3.1^{a} | 0^{a} |
| **Mean** | **6.29±0.33** | | | **5.11±0.42** | | **1.51±0.57** | |
| **RIN±SE** | | | | | | | |
| | | | | | | | |

| **Group C** | | | | | | | |
|---|---|---|---|---|---|---|---|
| | Cycle 0 | | | Cycle 3/4 | | Cycle 6/8 | |
| Patients (19) | Max RIN | % Tumour Cellularity | Her2 | Max RIN | % Tumour Cellularity | Max RIN | % Tumour Cellularity |
| CAMN004 | 5.9 | 100 | 0 | 1.8 | 100 | 2^{c} | 100^{c} |
| CAMN005 | 6.8 | 100 | 80 | 0 | 1 | 3.1^{a} | 0^{a} |
| CAMN009 | 7.1 | 90 | 0 | 2.7 | 70 | 0^{a} | 10^{a} |
| CAMN011 | 5.1 | 100 | 80 | 2.9 | 0 | 3^{a} | 0^{a} |
| CAMN012 | 6.4 | 90 | 80 | 0 | 0 | 0^{a} | 0^{a} |
| CAMN014 | 5.7 | 100 | 0 | 0 | 50 | 0 | |
| CAMN015 | 7.2 | 100 | 90 | 0 | 90 | 0^{a} | 10^{a} |
| CAMN016 | 7.1 | 100 | 0 | 0 | 0 | 2.9^{a} | 0^{a} |
| CAMN017 | 7.7 | 100 | 80 | 2.9 | 90 | 0^{a} | 0^{a} |
| CAMN020 | 7.4 | 100 | 0 | 0 | 5? | 0^{a} | 0^{a} |
| CAMN026 | 6.4 | 90 | 0 | 2.6 | 50 | 0^{c} | 30^{c} |
| CAMN029 | 5.8 | 100 | 80 | 0 | 90 | 0^{c} | 100^{c} |
| CAMN033 | 6.9 | 90 | 0 | 2.7 | 20 | 2.6^{a} | 0^{a} |
| CAMN034 | 7.7 | 70 | 0 | 2.5 | 50 | 2.8^{c} | 90^{c} |
| CAMN036 | 8.3 | 100 | 0 | 0 | 90 | 0^{c} | 50^{c} |
| CAMN038 | 8.6 | 100 | 80 | 0 | 0 | 3^{a} | 0^{a} |
| CAMN045 | 6.9 | 90 | 0 | 2.1 | 90 | 2.7^{a} | 5^{a} |
| CAGS001 | 5.7 | 80/100 | | 0 | 20 | 0^{a} | 5^{a} |
| CAGS002 | 7.1 | 80 | | 0 | 80 | 2.4^{c} | 50^{c} |
| **Mean RIN±SE** | **6.83±0.21** | | | **1.06±0.3** | | **1.29±0.32** | |
| | | | | | | | |

| **Group D** | | | | | | | |
|---|---|---|---|---|---|---|---|
| | Cycle 0 | | | Cycle 3/4 | | Cycle 6/8 | |
| Patients (5) | Max RIN | % Tumour Cellularity | Her2 | Max RIN | % Tumour Cellularity | Max RIN | % Tumour Cellularity |
| CAMN003 | 7.7 | 40 | 80 | 2.8 | 10 | 7.8^{b} | 50^{b} |
| CAMN008 | 6.1 | 50 | 80 | 2.7 | 100 | 6.5^{c} | 0^{c} |
| CAMN018 | 6.4 | 100 | 0 | 2.8 | 80 | 5.2^{b} | 90^{b} |
| CAMN032 | 4.7 | 100 | 0 | 0 | 100 | 5.9 | |
| CAMN047 | 7.2 | 60 | 0 | 2.4 | 0 | 8^{b} | 50^{b} |
| **Mean RIN±SE** | **6.42±0.52** | | | **2.14±0.54** | | **6.68±.54** | |

| **Group E** | | | | | | | |
|---|---|---|---|---|---|---|---|
| | Cycle 0 | | | | | Cycle 6/8 | |
| Patients (4) | Max RIN | % Tumour Cellularity | Her2 | | Cycle 3/4 | Max RIN | % Tumour Cellularity |
| CAMN013 | 0 | 60 | 0 | 0 | Max RIN | 0^{c} | 70^{c} |
| CAMN021 | 0 | 70 | 0 | 0 | 90 | 0^{c} | 50^{c} |
| CAMN027 | 2.8 | 100 | 80 | 2.9 | 0 | 0^{a} | 0^{a} |
| CAMN039 | 2.8 | 90 | 0 | 1.9 | 2 | 2^{a} | 0^{a} |
| **Mean RIN±SE** | **1.4±0.81** | | | **1.2±0.72** | | **0.5±0.5** | |

| | | | | | | | |
|---|---|---|---|---|---|---|---|
| Significant values are denoted as follows: ^{a} Reduction in maximum RIN post-treatment to ≤ 3.1 correctly identified responders in 19 of 20 cases (concordance was 95%); ^{b} Maximum tumour RIN values above 3.1 correctly identified non-responders in 16 of 25 cases (64% accuracy); ^{c} Discrepancy between maximum RIN and tumour cellularity. | | | | | | | |

### (g) Relationship of Tumour RNA Integrity and Clinical Response to Chemotherapy

The relationship between tumour RNA integrity and actual clinical response to disease was examined, as it was considered an important measure of the utility of tumour RNA integrity to serve as a biomarker of response to chemotherapy agents.

Patient response to chemotherapy treatment was measured in a variety of ways. Patients were deemed to have a complete clinical response (CR) if no tumours were evident following treatment. Tumours whose volume decreased by greater than 50% were deemed to have a partial response (PR) to therapy. Patients with tumours that exhibited no change in size were classified as having stable disease (SD), while patients with new tumours or whose tumours increased in size were said to have progressive disease (PD). Patients who had a complete resolution of disease microscopically were deemed to have exhibited a complete pathologic response (pCR). Using the above definitions of patient response to chemotherapy, the relationship between average or maximum tumour RNA values and clinical response to therapy was analyzed. Both average and maximum RIN values were computed, since one or both may have the greatest correlated with clinical response. In the MA.22 patients, 13 patients were observed to have CRs, while 37 patients were deemed nonresponders (PR, SD, or PD). Only 7 of the 50 patients exhibited a pCR. In particular, a low average tumour RIN post-treatment was significantly associated with a CR (p=0.01), while a low maximum RIN was associated with a pCR mid-treatment (p = 0.01), but not post-treatment (p = 0.28).

Figure 6 depicts the maximum tumour RIN value for each patient pre-, mid-, and post-treatment time. Patients that exhibited a pCR after epiribucin/docetaxel chemotherapy are shown with dashed lines. All patients that had a pCR post-chemotherapy exhibited a reduction in maximum tumour RIN mid-treatment. Moreover, both pCRs and reductions in tumour RINs were observed preferentially in patients exposed to high drug dose levels (levels 5 or higher).

It was noted that while a strong reduction in the maximum tumour RIN was observed mid-treatment for patients exhibiting pCRs post-therapy, tumour RIN values increased or stayed the same, despite complete resolution of disease microscopically. After initial decreases in RIN were observed mid-treatment, tumour RIN values increased post-treatment for a number of MA.22 patients. This may be due to the fact that since breast tumours are heterogeneous, the measured tumour RIN reflected the RNA quality of all cells comprising the tumour, including any normal breast tissue and other cell types. As shown in Table 4, the tumour cellularity of the vast majority of patient tumours pre-treatment was very high (90.94 ± 2.17).

As provided above, strongly reduced RIN values observed mid-treatment reflected a loss of RNA and RNA quality specifically in tumour cells. In view of the results, it would be expected that a low tumour RIN value would correlate with pCR mid-treatment. However, upon clearance of the chemotherapy drugs from the circulatory systems of patients post-treatment, it is possible that normal breast and other tissues may infiltrate the lesion in responders to therapy that no longer have detectable tumour cellularity. Hence, RNA isolated from lesions post-treatment may stem from both normal cells and tumour cells and a high tumour RIN value post-treatment may not necessarily indicate a recurrence of disease. This may explain why the relationship between low maximum tumour RIN values and pCR is most significant when patients are assessed mid-treatment. Furthermore, this may be why tumour RIN values post-treatment are more reliable at predicting responders to epirubicin/docetaxel chemotherapy than nonresponders. Should tumour biopsies post-treatment include non-tumour cells, which would be expected, then patients responding to epirubicin/docetaxel chemotherapy (in particular, a pCR) would be those patients that exhibit a strong reduction in tumour RIN mid- OR post-treatment.

### Example 3: Use of Tumour RNA Quality to Determine a Cancer Patient's Responsiveness to a Chemotherapy Regimen

According to the method of Example 1, RNA is extracted from tumour cells of a cancer patient with one or more tumours at two or more different time points during the administration of a chemotherapy regimen, before the administration of a chemotherapy regimen, and during and/or after completion of the regimen.

The tumour cells are collected in one or more image-guided biopsies. An image-guided biopsy is obtained with image-guided means such as computed tomography (CT), x-ray, ultrasound, and magnetic resonance imaging (MRI).

The quality of the extracted RNA is then determined by capillary electrophoresis of the extracted RNA and quantification of the RNAs in the resultant electropherogram. An automated analytical system, such as the Agilent® 2100 Bioanalyzer (Agilent Technologies, Inc., U.S.A.) is used for carrying out the RNA quality determination, in order to obtain an RNA integrity number (RIN) for each sample of RNA (Schroeder, A. et al., BMC. Mol. Biol. 7 (2006) 3; Imbeaud, S. et al. Nucl. Acids Res. (2005), 33, 6, e56, 1-12).

The RIN value of the tumour cells collected before administration of the chemotherapy regimen is then compared with the one or more RIN values of tumour cells collected after commencement of the regimen, i.e. during the regimen and/or after completion of the chemotherapy regimen. If a patient exhibits no change in tumour RNA integrity during treatment (response pattern A as noted in Example 2), then the patient's tumour would be considered resistant to the chemotherapy regimen being used. The patient would be considered at high risk of tumour progression and prognosis would be considered poor. Alternative chemotherapy regimens or treatment protocols should then be considered. The method outlined herein can be repeated to determine responsiveness to the new regimen.

If a patient exhibits a dramatic reduction in tumour RNA integrity (>50%) both mid- and post-treatment (response pattern C as noted in Example 2), then the patient would be considered to have responded to chemotherapy and would be at lower risk of tumour progression. The patient's prognosis would be considered good. Tumour RIN values near zero would be highly indicative of response to chemotherapy and low risk of tumour progression.

If a patient exhibits a dramatic reduction in tumour RNA integrity post-treatment only (response pattern B as noted in Example 2), then the patient would be considered to have responded to chemotherapy and be at lower risk of tumour progression. The patient's prognosis would be considered good.

If a patient exhibits a dramatic change in tumour RNA integrity mid-treatment only, then she likely has responded to therapy and would be at a lower risk of disease recurrence. This is regardless of a return to high "tumour" RNA integrity post-treatment, since the high quality RNA post-treatment may stem from normal tissue that has infiltrated the lesion. However, in this case, it is possible that the tumour has recurred post-treatment.

## Claims

1. A method of determining tumour responsiveness to a chemotherapeutic agent in a sample comprising tumour cell RNA, obtained from a patient with a cancerous tumour after the patient has received one or more doses of the chemotherapeutic agent, comprising determining the RNA integrity assessed by degradation of the sample and comparing the RNA integrity of the sample to a time point before administration of the one or more doses of the chemotherapeutic agent;
wherein a decrease in RNA integrity over time is indicative that the tumour is responsive to the chemotherapeutic agent and a high RNA integrity of the sample, wherein the extracted RNA exhibits little to no degradation, is indicative that the tumour is resistant to the chemotherapeutic agent.

2. The method of claim 1, wherein the RNA integrity is determined by: calculating a 28S:18S ribosomal RNA (rRNA) ratio; or using i) capillary electrophoresis, and ii) quantification of various RNAs separated in the electropherogram.

3. The method of claim 1 or 2, wherein the RNA integrity is expressed as an RNA integrity number (RIN), wherein the RIN comprises a calculation of RNA integrity of multiple RNAs.

4. The method of any one of claims 1 to 3, wherein the sample is a post-chemotherapy regimen sample and/or obtained during a chemotherapy regimen.

5. The method of claim 3 or 4, wherein a RIN of 3 or less is indicative that the tumour is responsive to the chemotherapeutic agent.

6. The method of any one of claims 3, 4 or 5, wherein a RIN of more than 3 is indicative that the tumour is resistant to the chemotherapeutic agent.

7. The method of any one of claims 1 to 6, wherein the sample comprises tumour cells or a tumour biopsy.

8. The method according to claim 7 wherein the cancerous tumour is breast cancer.

9. The method of any one of claims 1 to 8, wherein the chemotherapeutic agent is selected from one or more of anthracyclines, taxanes and combinations thereof.

10. The method of claim 9 wherein the chemotherapeutic agent comprises epirubicin and docetaxel.

11. The method of any one claims 1 to 10, wherein the tumour cell RNA is isolated.

12. The method of any one of claims 1 to 11, wherein the patient is part of a clinical trial.

13. A method of determining a patient's responsiveness to a chemotherapeutic agent, in a patient with a cancerous tumour, comprising determining a RNA integrity assessed by degradation of RNA in a sample of tumour cells obtained from the patient before administration of the chemotherapeutic agent, and comparing with the RNA integrity of tumour cells determined after administration of the chemotherapeutic agent, wherein a decrease in the RNA integrity after administration of the chemotherapeutic agent indicates that the patient is responsive to the chemotherapeutic agent.

14. A method of tailoring a chemotherapy treatment in a patient with a cancerous tumour comprising determining tumour responsiveness to a chemotherapeutic agent according to any one of claims 1 to 13, wherein a decrease in the RNA integrity assessed by degradation of RNA in the sample provides an indication that the chemotherapy should continue and wherein an absence of a decrease in the RNA integrity of the sample provides an indication that the cancer treatment should be altered, for example by altering the dosage level and/or changing the chemotherapy agent.

## Patentansprüche

1. Verfahren zur Bestimmung des Ansprechens eines Tumors auf ein Chemotherapeutikum in einer Tumorzellen-RNA umfassenden Probe, die von einem Patienten mit einem Krebstumor gewonnen wurde, nachdem der Patient eine oder mehr Dosen des Chemotherapeutikums erhalten hat, umfassend das Bestimmen der anhand der Degradation der Probe bewerteten RNA-Integrität und das Vergleichen der RNA-Integrität der Probe mit einem Zeitpunkt vor der Verabreichung der einen oder mehreren Dosen des Chemotherapeutikums;
wobei eine Abnahme der RNA-Integrität im Zeitverlauf anzeigt, dass der Tumor auf das Chemotherapeutikum anspricht, und eine hohe RNA-Integrität der Probe, wobei die extrahierte RNA wenig bis keine Degradation zeigt, anzeigt, dass der Tumor resistent gegen das Chemotherapeutikum ist.

2. Verfahren nach Anspruch 1, wobei die RNA-Integrität bestimmt wird durch: Berechnung eines 28S:18S ribosomalen RNA-Verhältnisses (rRNA), oder Verwendung von i) Kapillarelektrophorese und ii) Quantifizierung verschiedener im Elektropherogramm getrennter RNAs.

3. Verfahren nach Anspruch 1 oder 2, wobei die RNA-Integrität als RNA-Integritätsnummer (RIN) ausgedrückt wird, wobei die RIN eine Berechnung der RNA-Integrität mehrerer RNAs aufweist.

4. Verfahren nach einem der Ansprüche 1 bis 3, wobei die Probe eine postchemotherapeutische Probe ist und/oder während einer chemotherapeutischen Kur gewonnen wurde.

5. Verfahren nach Anspruch 3 oder 4, wobei eine RIN von 3 oder weniger anzeigt, dass der Tumor auf das Chemotherapeutikum anspricht.

6. Verfahren nach einem der Ansprüche 3, 4 oder 5, wobei eine RIN von mehr als 3 anzeigt, dass der Tumor resistent gegen das Chemotherapeutikum ist.

7. Verfahren nach einem der Ansprüche 1 bis 6, wobei die Probe Tumorzellen oder eine Tumorbiopsie umfasst.

8. Verfahren nach Anspruch 7, wobei der Krebstumor Brustkrebs ist.

9. Verfahren nach einem der Ansprüche 1 bis 8, wobei das Chemotherapeutikum aus einem oder mehreren von Anthracyclinen, Taxanen und deren Kombinationen gewählt wird.

10. Verfahren nach Anspruch 9, wobei das Chemotherapeutikum Epirubicin und Docetaxel umfasst.

11. Verfahren nach einem der Ansprüche 1 bis 10, wobei die Tumorzellen-RNA isoliert wird.

12. Verfahren nach einem der Ansprüche 1 bis 11, wobei der Patient Teil eines klinischen Versuchs ist.

13. Verfahren zur Bestimmung des Ansprechens eines Patienten auf ein Chemotherapeutikum bei einem Patienten mit einem Krebstumor, umfassend das Bestimmen einer RNA-Integrität, bewertet anhand einer Degradation der RNA in einer Probe von Tumorzellen, die von dem Patienten vor Verabreichung des Chemotherapeutikums gewonnen wurde, und Vergleichen mit der RNA-Integrität der Tumorzellen, die nach Verabreichung des Chemotherapeutikums bestimmt wurde, wobei eine Abnahme der RNA-Integrität nach Verabreichung des Chemotherapeutikums anzeigt, dass der Patient auf das Chemotherapeutikum anspricht.

14. Ein Verfahren zur individuellen Anpassung einer chemotherapeutischen Behandlung bei einem Patienten mit einem Krebstumor, umfassend das Bestimmen des Ansprechens eines Tumors auf ein Chemotherapeutikum nach einem der Ansprüche 1 bis 13, wobei eine Abnahme der durch Degradation der in der Probe enthaltenen RNA bewerteten RNA-Integrität darauf hindeutet, dass die Chemotherapie fortgesetzt werden sollte, und wobei das Ausbleiben einer Abnahme der RNA-Integrität der Probe darauf hindeutet, dass die Krebsbehandlung geändert werden sollte, zum Beispiel durch Änderung des Dosierungsgrads und/oder Wechsel des Chemotherapeutikums.

## Revendications

1. Procédé de détermination de la réponse d'une tumeur à un agent chimiothérapeutique dans un échantillon comprenant l'ARN de cellules tumorales, obtenu auprès d'un patient présentant une tumeur cancéreuse après que le patient a reçu une ou plusieurs doses de l'agent chimiothérapeutique, comprenant la détermination de l'intégrité de l'ARN évaluée par dégradation de l'échantillon et la comparaison de l'intégrité de l'ARN de l'échantillon à un point de temps avant l'administration de la ou des doses de l'agent chimiothérapeutique ;
dans lequel une diminution de l'intégrité de l'ARN dans le temps indique que la tumeur répond à l'agent chimiothérapeutique et une intégrité élevée de l'ARN de l'échantillon, dans lequel l'ARN extrait exhibe peu ou pas de dégradation, indique que la tumeur est résistante à l'agent chimiothérapeutique.

2. Procédé selon la revendication 1, dans lequel l'intégrité de l'ARN est déterminée par : calcul d'un rapport d'ARN ribosomaux (ARNr) 28S:18S ; ou utilisation i) d'une électrophorèse capillaire, et ii) d'une quantification de divers ARN séparés dans l'électrophérogramme.

3. Procédé selon la revendication 1 ou 2, dans lequel l'intégrité de l'ARN est exprimée sous forme d'indice d'intégrité de l'ARN (RIN), dans lequel le RIN comprend un calcul de l'intégrité de l'ARN de multiples ARN.

4. Procédé selon l'une quelconque des revendications 1 à 3, dans lequel l'échantillon est un échantillon obtenu après une chimiothérapie et/ou obtenu pendant une chimiothérapie.

5. Procédé selon la revendication 3 ou 4, dans lequel un RIN inférieur ou égal à 3 indique que la tumeur répond à l'agent chimiothérapeutique.

6. Procédé selon l'une quelconque des revendications 3, 4 ou 5, dans lequel un RIN supérieur à 3 indique que la tumeur est résistante à l'agent chimiothérapeutique.

7. Procédé selon l'une quelconque des revendications 1 à 6, dans lequel l'échantillon comprend des cellules tumorales ou une biopsie tumorale.

8. Procédé selon la revendication 7, dans lequel la tumeur cancéreuse est un cancer du sein.

9. Procédé selon l'une quelconque des revendications 1 à 8, dans lequel l'agent chimiothérapeutique est choisi parmi un ou plusieurs des anthracyclines, des taxanes et de combinaisons de ceux-ci.

10. Procédé selon la revendication 9, dans lequel l'agent chimiothérapeutique comprend l'épirubicine et le docétaxel.

11. Procédé selon l'une quelconque des revendications 1 à 10, dans lequel l'ARN d'une cellule tumorale est isolé.

12. Procédé selon l'une quelconque des revendications 1 à 11, dans lequel le patient fait partie d'un essai clinique.

13. Procédé de détermination de la réponse d'un patient à un agent chimiothérapeutique, chez un patient présentant une tumeur cancéreuse, comprenant la détermination d'une intégrité de l'ARN évaluée par dégradation de l'ARN dans un échantillon de cellules tumorales obtenu auprès du patient avant l'administration de l'agent chimiothérapeutique et la comparaison de l'intégrité de l'ARN des cellules tumorales déterminée après l'administration de l'agent chimiothérapeutique, dans lequel une diminution de l'intégrité de l'ARN après l'administration de l'agent chimiothérapeutique indique que le patient répond à l'agent chimiothérapeutique.

14. Procédé d'adaptation d'un traitement chimiothérapeutique chez un patient présentant une tumeur cancéreuse, comprenant la détermination de la réponse de la tumeur à un agent chimiothérapeutique selon l'une quelconque des revendications 1 à 13, dans lequel une diminution de l'intégrité de l'ARN évaluée par dégradation de l'ARN dans l'échantillon indique que la chimiothérapie devrait être poursuivie et dans lequel une absence de diminution de l'intégrité de l'ARN de l'échantillon indique que le traitement anti-cancéreux devrait être modifié, par exemple en modifiant le taux du dosage et/ou en changeant l'agent chimiothérapeutique.
